# EUROPEAN PATENT SPECIFICATION

(11) **EP 0 605 395 B1**
(45) Date of publication and mention of the grant of the patent: **02.04.2003**
(21) Application number: 94101963.0
(22) Date of filing: 18.01.1991
(51) Int. Cl.: A61M 1/16, B01F 5/10

(54) **Apparatus and method for preparation of a fluid intended for medical use**
Vorrichtung und Verfahren zur Herstellung einer für medizinische Zwecke vorgesehenen Flüssigkeit
Dispositif et procédé pour la préparation d'un fluide à usage médicale

(30) Priority: 19.02.1990 SE 9000586
(43) Date of publication of application: 06.07.1994
(62) Divisional of application: 91100559.3
(73) Proprietor: GAMBRO AB, 220 10 Lund (SE)
(72) Inventor: Jönsson, Lennart, S-240 20 Furulund (SE); Knutsson, Stefan, S-237 00 Bjärred (SE)
(74) Representative: Asketorp, Göran

(56) References cited:
- EP-A- 0 278 100
- EP-A- 0 401 130
- DE-A- 2 924 406
- FR-A- 1 333 222
- US-A- 4 082 667
- US-A- 4 734 198
- US-A- 4 848 916

## Description

### TECHNICAL FIELD

The present invention relates to a method and apparatus for preparation of a fluid intended for medical use, for example, dialysis fluid or replacement fluid for hemofiltration or a concentrate for preparation of such fluids, including a source of pure water and a mixing vessel containing a powder which is to be dissolved in the said water for preparation of the desired fluid.

### BACKGROUND ART

Previous systems available for similar purposes normally start from one or more liquid based concentrates. For example US-A-4 158 034, describes how a concentrate in liquid form is mixed with water for preparation of a dialysis fluid. EP-A-0 022 922 and US-A-4 783 273 describe how two liquid-based concentrates are mixed with water to achieve a fluid intended for medical use, for example dialysis fluid. More recent systems make use of one or more concentrates in powder form. As an example, US-A-4 784 495 describes how one or more such powder-based concentrates can be used with or without addition of a liquid-based concentrate. This system prepares the fluid in a continuous manner.

US-A-4 848 916 describes a powder based system for bulk production of concentrates in a batch wise manner. A predetermined amount of powder to be dissolved and a predetermined amount of water are mixed by recirculating the water until the powder is completely dissolved. Thus a large "bulk" quantity of uniform concentrate can be prepared.

### DISCLOSURE OF THE INVENTION

The above-mentioned problems are solved by the present invention which thus relates to a method and apparatus for preparation of a fluid intended for medical use, for example dialysis fluid or replacement fluid for hemofiltration or a concentrate for preparation of such fluids, including a source of pure water and a mixing vessel containing a powder which is to be dissolved in the said water for preparation of the desired fluid as defined in method claim 1 apparatus claim 10.

Preferably the means for recirculating the water or partially prepared fluid consists of a recirculation pump of a type such that it is suitable for metering the fluid prepared in the mixing vessel.

To control the condition of the mixture, a conductivity measuring device is appropriately arranged in the recirculation circuit, said device being arranged so as to stop the recirculation through the connected mixing vessel when the desired conductivity is obtained. Other suitable means for measuring the concentration, such as a pH-meter or an ion-selective meter, can, of course, be used as well. The same substitution can also be made for the conductivity meters mentioned in the rest of this description.

To control the quantity of water supplied to said mixing vessel a valve is suitably arranged in the transport conduit between the water source and this vessel, the valve being arranged to be closed when the mixing vessel contains a predetermined quantity of water or partially prepared fluid. The mixing vessel can for example comprise a level indicator which controls the valve arranged in the transport conduit.

Said vessel should appropriately contain from the outset an excess of powder concentrate, so that the circulation in the recirculation circuit can be interrupted before the vessel connected to the recirculation circuit is totally emptied. Hereby it is assured that the desired concentration of the added substance can always be obtained.

Certain advantages can be obtained if the mixing vessel in addition to the said salt also contains a smaller quantity of liquid, preferably water, of such quantity that a concentrated liquid or mud of said liquid and the powder-based salt is formed. Such a concentrated liquid can more easily be checked that it is lump-free compared with a more or less compacted powder in dry form.

The salt in the form of dry powder or concentrated liquid in the mixing vessel is more easily dissolved when the recirculation circuit includes a heating device.

Particularly when the mixing vessel contains a concentrated liquid, besides the said salt, it can also contain an acid in liquid form. Many medical solutions, for example dialysis fluid, should actually contain such an acid and this can already be added before the preparation of the final solution. Alternatively the acid can be added by means of the recirculation circuit being connected to a branch conduit with a dosage-pump, which is arranged to pump an acid in liquid-form from its storage vessel to the recirculation circuit. From a medical point of view it can be appropriate for the recirculation circuit to include a sterile filter, which is preferably arranged in the circulation direction just before the mixing vessel.

According to a preferred embodiment of the invention the mixing vessel is connected to the recirculation circuit with the help of quick-released connectors so thus it can easily be uncoupled and connected instead to, for example, a dialysis machine instead of the traditionally employed dialysis concentrate container.

The mixing vessel can appropriately consist of a flexible plastic bag. By the use of such a bag a check can be made to see if the salt is lump-free, whether it is in dry form or in the form of a liquid concentrate.

### BRIEF DESCRIPTION OF THE DRAWINGS

Figures 1-3 which are not according to the invention show in the form of a block diagram an environment for the invention in three different connectable positions.
Fig. 4 which is not according to the invention shows a diagram of the measured conductivity in the above-described recirculation circuit with the supply of three different concentrates.
Figures 5a and 5b finally show a preferred embodiment of the system according to the invention.

### PREFERRED EMBODIMENTS OF THE SYSTEM ACCORDING TO THE INVENTION

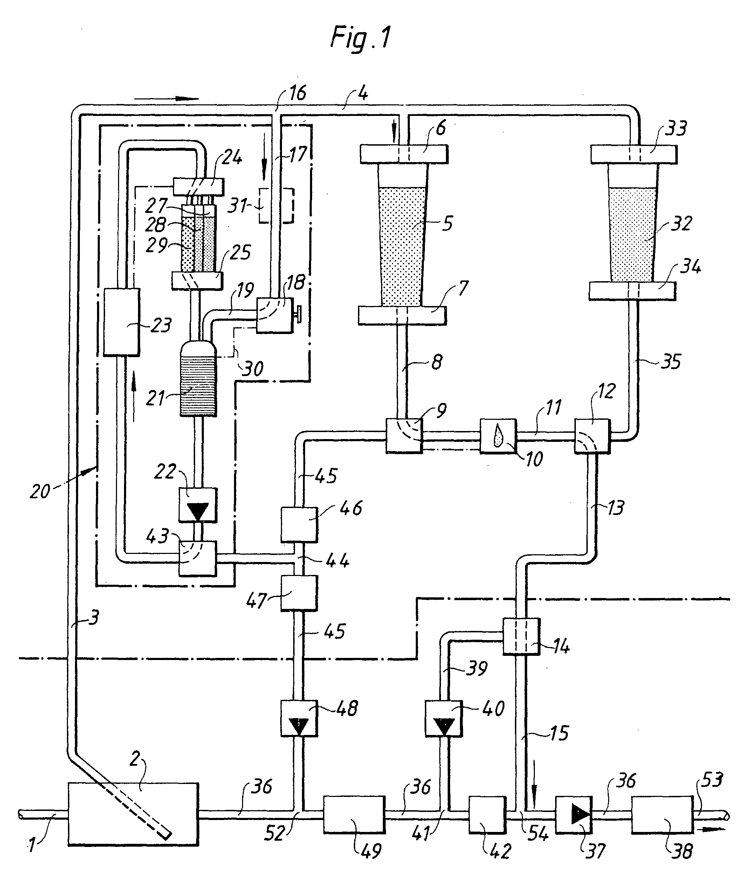

Figures 1-3 which are not according to the invention show by way of a block diagram an environment for the invention in three different connectable positions. Figure 1 is hereby intended to show how a recirculation circuit 20, respectively a separate powder cartridge 5, is filled with water. This water is taken from an inlet 1 via a heating vessel 2 through conduits 3 and 4 to said powder cartridge 5, which is located between two connection pieces 6 and 7. When the cartridge 5 is filled, the water is further passed as showed in figure 1 through a conduit 8 and a valve 9 to a detector 10, which detects the water and/or concentrate dissolved therein. This can possibly occur with a certain time delay so that the fluid will have time to fill the subsequent conduit 11, valve 12, conduit 13, valve 14 and conduit 15.

At the same time, water is conveyed from a branch point 16 through a conduit 17, a valve 18 and a conduit 19 to a recirculation circuit, which in its entirety is denoted by 20. This circuit includes a mixing vessel 21, a recirculation pump 22, a concentration measuring device 23, for example a conductivity meter, and connected between two valve units 24 and 25 a number of cartridges or other vessels 27, 28 and 29 for one or more powder concentrates and possibly some liquid-based concentrate. When a sufficient quantity fluid is supplied to the mixing vessel 21, the valve 18 is closed. This can occur with the help of a level indicator schematically shown by the dashed line 30. Thereafter, the recirculation pump 20 is started with the valve units 24 and 25 so positioned that the water is passed through one of the vessels or cartridges 27-29. In figure 1 it is the vessel 29 which is connected. When the desired conductivity is obtained, which is controlled with the help of the meter 23, the next cartridge 28 is instead connected, and then finally cartridge 27. Should the prepared fluid in the mixing vessel 21 include another liquid-based or easily dissolvable concentrate, then the dissolving can occur during the filling of the mixing vessel 21 with the vessel containing this concentrate being connected to the conduit 17. Such a vessel 31 is indicated in a block-form with the help of dashed lines.

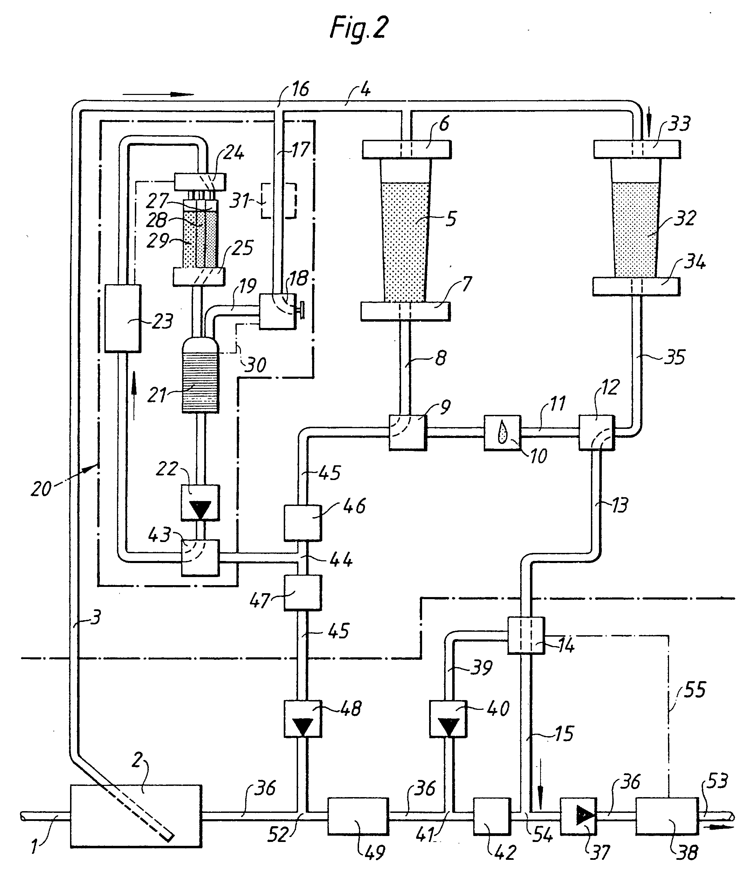

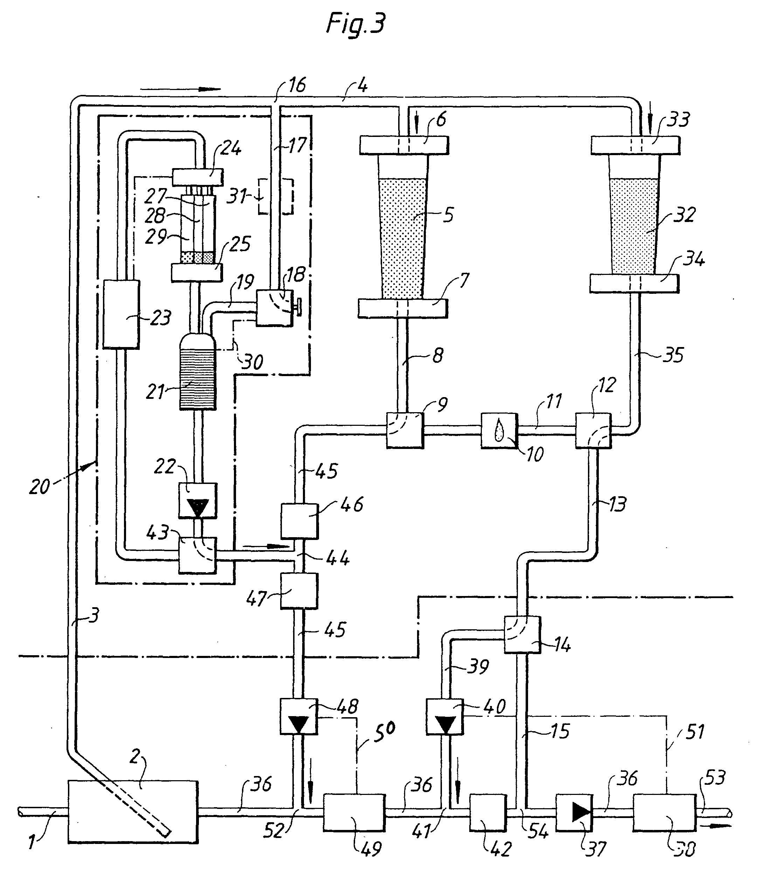

When the recirculation circuit 20 is filled, the valve 18 is closed as showed in figure 2. In the same way, valve 9 is actuated when the vessel 5 is filled. This position is also showed in figure 2. At the same time, the valve 12 is switched from the position shown in figure 1 to that in figure 2, so that water from the conduit 4 can pass to another vessel or powder cartridge 32 located between two connection pieces 33 and 34. When this cartridge 32 has been filled the water with dissolved concentrate therein is conducted through the conduit 35, valve 12, conduit 13, valve 14 and conduit 15 to a main line or conduit 36 which includes a pump 37 and a detector 38, for example a conductivity measuring device. This detector controls the valve 14 so that it is switched to the position shown in figure 3. The fluid from the vessel 32 is now conveyed instead from the valve 14 via a conduit 39 with a dosage pump 40 to the main line 36 to a point 41 upstream of a restrictor 42. This restrictor 42, together with the pump 37 and a gastrap not shown arranged further downstream, is used for de-gasing the prepared fluid.

With the switching of the valve 14 to the position shown in figure 3, the described system is ready for dialysis. An inlet valve 43 in the recirculation circuit 20 is thus actuated so the pump 22 can pump prepared fluid from the mixing vessel 21 to a mixing point 44, to which concentrate from the vessel 5 is conducted via the valve 9 and the conduit 45. The appropriate concentration of the dissolved substance is measured in this conduit upstream and downstream of the mixing point 44. This can be achieved, for example, by means of a differential conductivity meter, whose measuring points are denoted by 46 and 47 respectively. The prepared solution can then be conducted further through the conduit 45 with the help of a pump 48, which preferably consists of an accurately metering dosage pump. With the help of this the prepared solution is conducted to the main line 36, in which the concentration is once more checked by means of a meter 49, for example another conductivity measuring device. This meter 49 then controls the pump 48, as showed by the dashed line 50 in figure 3. Additional concentrate is then added to the prepared solution at the mixing point 41 by means of a pump 40 controlled by the measuring device 38 as indicated by the dashed line 51. By means of this measuring device 38, which preferably consists of a conductivity meter, the final concentration of the prepared solution obtained from the system according to the invention is regulated.

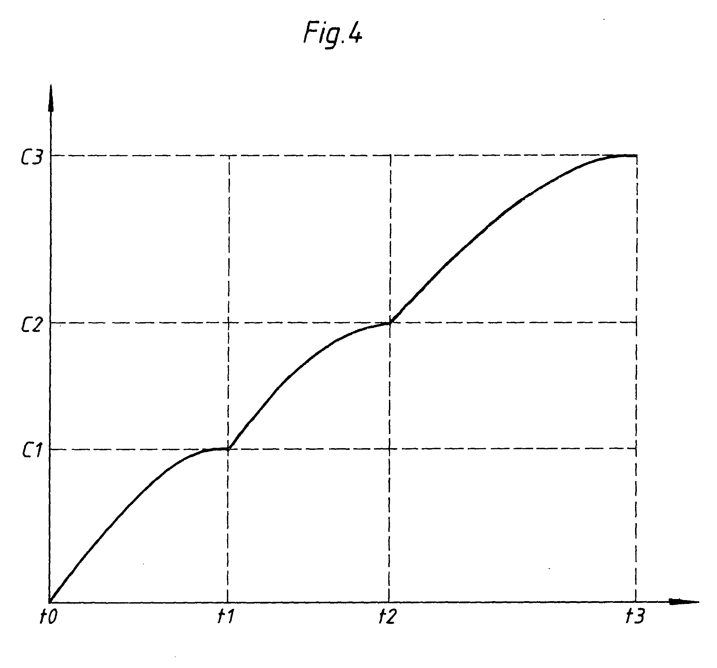

Finally, figure 4 which is not according to the invention shows a diagram of how the conductivity in the recirculation circuit 20 varies. Here it is assumed that the meter 23 consists of a conductivity measuring device and that the vessels 27, 28 and 29 contain three different salts. Thus, between time t0 and t1 one of these vessels is connected until the conductivity value c1 is obtained. Thereafter, the next vessel is connected until the conductivity value c2 is reached. Then the last vessel is connected which remains connected until the conductivity obtains the desired value c3. At this point the valve 43 is switched over so that the ready-prepared solution can be conducted to the mixing point 44. At this mixing point concentrate from the vessel 5 is added. The thus obtained mixture is then led to a mixing point 52 in the main line 36 where it is mixed with water from the heating vessel 2. At the next mixing point 41, concentrate from the vessel 32 is then added. After a final check in the measuring device 38, the ready prepared solution can then be conducted to its place of consumption, as symbolized by an arrow 53.

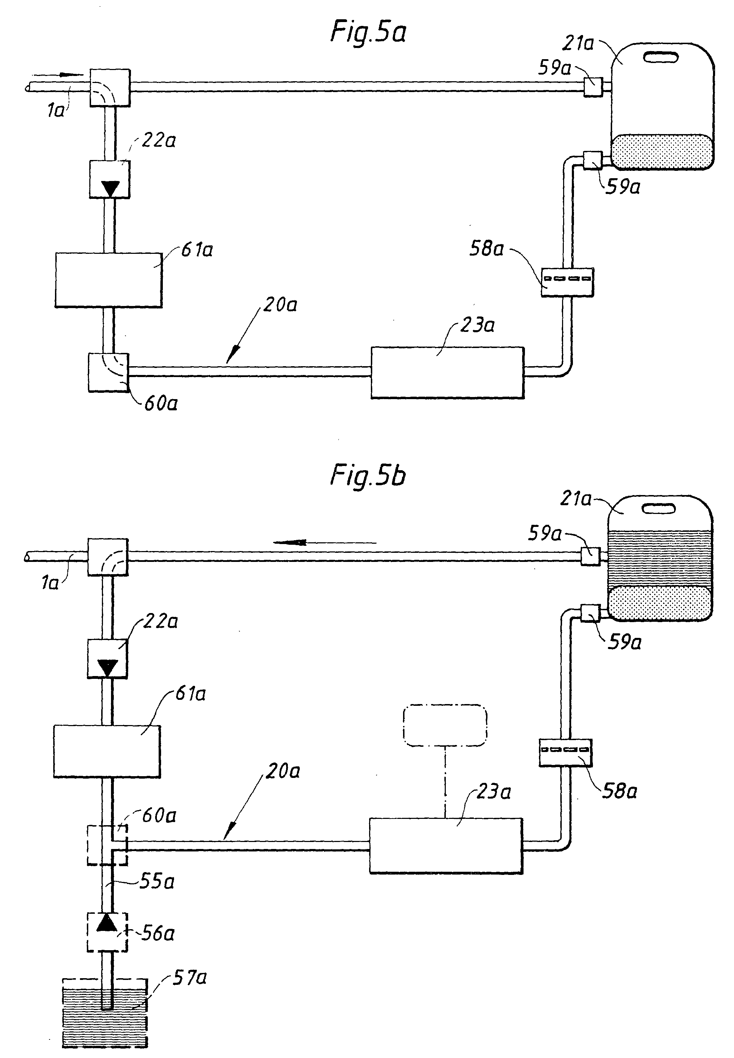

Finally figures 5a and 5a show a preferred embodiment of the apparatus according to the invention. Since this modification corresponds in principal with the above-described system, the same reference numerals have been used, but with the addition of a. In this system, pure water enters via an inlet 1a and is pumped with the help of a pump 22a through a recirculation circuit 20a which further includes a heater 61a, a control device, for example a conductivity meter 23a, a sterile filter 58a and a mixing vessel 21a. With this arrangement the mixing vessel 21a should, from the outset, contain the desired quantity salt. This can possibly be in dry form, or as a concentrated liquid obtained by adding a small quantity of liquid thereto. Such a concentrated liquid can be complemented by possibly necessary acid in liquid form. This acid may however also be added in different forms. Certain acids can, for example, be obtained in dry powder-form.

Acid in liquid form can be added via a branch conduit 55a with a dosage pump 56a, as shown in figure 5b. The acid can thus be taken from a vessel 57a and introduced into the recirculation circuit via a valve 60a. The mixing vessel 21a is preferably connected to the recirculation circuit 20a via quick-release connectors 59a, so that it can easily be uncoupled and connected instead to, for example, a dialysis machine instead of the traditionally employed dialysis concentrate container.

### EXAMPLE

Using the system described in figure 5a and 5b for preparation of a dialysis liquid solution, the following mixture was presence in the vessel 21a:
NaCl ≈ 1 050 gram
KCl ≈ 13 gram
CaCl ≈ 45 gram
MgCl ≈ 18 gram.

All of these salts were present in the form of a powder. To this was added approximately 2 dl of fluid, comprising essentially water, but to which approximately 32 g of acetic acid had been added. In this case a flexible plastic bag was used for the mixing vessel 21a. Thereby it was possible to check that a lump-free concentrated liquid was obtained. It should be appreciated that a somewhat smaller quantity of liquid can be used. Alternatively an excess of liquid can be added, but for practical reasons it is advisable to keep the weight of the mixing vessel 21 and its contents down.

Naturally the invention is not limited simply to the embodiment described above, but may be varied within the scope of the following claims. For example, the parts included in the system may be varied within wide limits with regard to their form as well as their function. For example the containers 27-29 and 31 can be combined together, for example in the form of a flexible bag or similar with a desired number of compartments.

## Claims

1. Method for preparation of a fluid intended for medical use, for example dialysis fluid or replacement fluid for hemofiltration or a concentrate for preparation of such fluids, **comprising** the steps of:
- providing a source (1a) of water and a mixing vessel (21a) containing a powder which is to be dissolved in said water for preparation of the desired fluid, means for conducting the water to said mixing vessel (21a), a recirculation circuit (20a) including this mixing vessel (21a) and means (22a) for recirculation of the water or partially prepared fluid through said mixing vessel (21a), supplying water from said source (1a), after ending supplying water from said source (1a) recirculating the water or partially prepared fluid through said mixing vessel, measuring (23a) the conductivity of the water or partially prepared fluid in said recirculation circuit, and stopping the recirculation when an appropriate concentration is reached from the complete or partial dissolving of the powder.

2. A method according to claim 1, further comprising the step of filling the mixing vessel with an excess of powder concentrate, preferably in excess of the amount required for a plurality of individual patient doses.

3. A method according to any one of the previous claims, further comprising the step of filtering the water or partially prepared fluid in the recirculation circuit.

4. A method according to any one of the previous claims, further comprising the step of supplying acid to said recirculation circuit (20a).

5. A method according to any one of the previous claims, wherein said powder is in dry form.

6. A method according to claims 1 to 4, wherein said powder is in the form of a concentrated liquid or mud obtained by adding a small quantity of liquid.

7. A method according any of the previous claims, wherein said powder comprising an acid in liquid or dry powder form.

8. A method according to any one of the previous claims, further comprising the step of uncoupling the mixing vessel (21a) from the recirculation circuit in order to connect the mixing vessel (21a) to a dialyses machine.

9. A method according any one of the previous claims, further **comprising** the step of recirculating said partially prepared solution from a first fluid connection of said mixing vessel, via the recirculation conduit to a second fluid connection of said mixing vessel, whereby said first fluid connection is arranged above said second fluid connection.

10. Apparatus for preparation of a fluid intended for medical use, for example dialysis fluid or replacement fluid for hemofiltration or a concentrate for preparation of such fluids, including a source (1a) of water and a mixing vessel (21a) containing a powder which is to be dissolved in said water for preparation of the desired fluid, comprising means for conducting the water to said mixing vessel (21), a recirculation circuit (20a) including this mixing vessel (21a), a valve for disconnecting the recirculation circuit from said source of water, means (22a) for recirculation of the water or partially prepared fluid through said mixing vessel (21a) and means for measuring the concentration within the recirculation circuit (20), for terminating the recirculation when an appropriate concentration is obtained from the complete or partial dissolving of the powder.

11. Apparatus according to claim 10, **wherein** the mixing vessel also contains a small quantity of a liquid, preferably water, in such a quantity that a concentrated liquid of said liquid and the powder is formed.

12. Apparatus according to claim 10 or 11, **wherein** the mixing vessel (21a) contains a liquid acid as well as said powder.

13. Apparatus according to any of claims 10-12, **wherein** the recirculation circuit (20a) includes a heating device (54a).

14. Apparatus according to any of claims 10-13, **wherein** the recirculation circuit (20a) is connected to a branch conduit (54a) with a dosage pump (56a), which is arranged to pump an acid in liquid form from a storage vessel (57a) to the recirculation circuit.

15. Apparatus according to any of claims 10-14, **wherein** the recirculation circuit (20a) includes a sterile filter (58a) which is preferably arranged in the circulation direction just before the mixing vessel (21a).

16. Apparatus according to any of claims 10-15, **wherein** the mixing vessel (21a) is connected to the recirculation circuit with the help of quick-release connectors (59a) so that it can easily be uncoupled and connected instead to, for example, a dialysis machine instead of the traditionally employed dialysis concentrate container.

17. Apparatus according to any of claims 10-16, **wherein** the mixing vessel (21a) consists of a flexible plastic bag or a cartridge.

18. Apparatus according to any of claims 10-17, **wherein** said means for measuring said concentration comprises a conductivity meter (23a).

19. Apparatus according to any of claims 10-18, **wherein** said recirculation circuit being connected to a first fluid connection (59a), of said mixing vessel (21a) for withdrawing the water or partially prepared fluid from said mixing vessel and to a second fluid connection (59a) of said mixing vessel (21a) for returning the water or partially prepared fluid to said mixing vessel, whereby said first fluid connection is arranged above said second fluid connection.

20. Apparatus according to any of the previous claims, **wherein** the mixing vessel (21a) for preparation of a dialysis fluid contains an amount in the magnitude 1 kg of powder mixture of suitable, for example conventional, composition to which an amount in the magnitude of 2 dl of a liquid is added for preparation of a lump-free concentrated liquid or slush, which preferably contains the necessary quantity of acid for the dialysis fluid.

## Patentansprüche

1. Verfahren zur Herstellung einer für medizinische Zwecke vorgesehenen Flüssigkeit, zum Beispiel einer Dialyseflüssigkeit oder einer Ersatzflüssigkeit für die Hämofiltration oder eines Konzentrates zur Herstellung von solchen Flüssigkeiten, umfassend die Schritte:
dass eine Wasserquelle (1a) und ein Mischgefäß (21a), das ein Pulver enthält, welches in dem Wasser zur Herstellung der gewünschten Flüssigkeit aufzulösen ist, ein Mittel zur Leitung des Wassers zu dem Mischgefäß (21a), ein Umwälzkreis (20a) umfassend dieses Mischgefäß (21a) und ein Mittel (22a) zur Umwälzung des Wassers oder der teilweise hergestellten Flüssigkeit durch das Mischgefäß (21a) vorgesehen werden,
dass Wasser von der Quelle (1a) zugeführt wird,
dass nach der Beendigung der Zufuhr von Wasser von der Quelle (1a) das Wasser oder die teilweise hergestellte Flüssigkeit durch das Mischgefäß umgewälzt wird,
dass die Leitfähigkeit des Wassers oder der teilweise hergestellten Flüssigkeit in dem Umwälzkreis gemessen (23a) wird, und
dass die Umwälzung angehalten wird, wenn eine entsprechende Konzentration durch eine vollständige oder teilweise Auflösung des Pulvers erreicht ist.

2. Verfahren nach Anspruch 1, weiter umfassend den Schritt, dass das Mischgefäß mit einem Überschuss an Pulverkonzentrat gefüllt wird, vorzugsweise die für eine Vielzahl von einzelnen Patientendosen erforderliche Menge übersteigend.

3. Verfahren nach einem der vorhergehenden Ansprüche, weiter umfassend den Schritt, dass das Wasser oder die teilweise hergestellte Flüssigkeit in dem Umwälzkreis gefiltert wird.

4. Verfahren nach einem der vorhergehenden Ansprüche, weiter umfassend den Schritt, dass Säure zu dem Umwälzkreis (20a) zugeführt wird.

5. Verfahren nach einem der vorhergehenden Ansprüche, worin das Pulver in trockener Form vorliegt.

6. Verfahren nach den Ansprüchen 1 bis 4, worin das Pulver in der Form einer konzentrierten Flüssigkeit oder Aufschlämmung vorliegt, die man durch Zugabe einer geringen Menge an Flüssigkeit erhält.

7. Verfahren nach einem der vorhergehenden Ansprüche, worin das Pulver eine Säure in flüssiger oder trockener Pulverform umfasst.

8. Verfahren nach einem der vorhergehenden Ansprüche, weiter umfassend den Schritt, dass das Mischgefäß (21a) von dem Umwälzkreis abgekoppelt wird, um das Mischgefäß (21a) an eine Dialysemaschine anzuschließen.

9. Verfahren nach einem der vorhergehenden Ansprüche, weiter umfassend den Schritt, dass die teilweise hergestellte Lösung von einem ersten Flüssigkeitsanschluss des Mischgefäßes über den Umwälzkanal zu einem zweiten Flüssigkeitsanschluss des Mischgefäßes umgewälzt wird, wobei der erste Flüssigkeitsanschluss über dem zweiten Flüssigkeitsanschluss angeordnet ist.

10. Vorrichtung zur Herstellung einer für medizinische Zwecke vorgesehenen Flüssigkeit, zum Beispiel einer Dialyseflüssigkeit oder einer Ersatzflüssigkeit für die Hämofiltration oder eines Konzentrates zur Herstellung von solchen Flüssigkeiten, mit einer Wasserquelle (1a) und einem Mischgefäß (21a), das ein Pulver enthält, welches in dem Wasser zur Herstellung der gewünschten Flüssigkeit aufzulösen ist, umfassend ein Mittel zur Leitung des Wassers zu dem Mischgefäß (21), einen Umwälzkreis (20a) umfassend dieses Mischgefäß (21a), ein Ventil zur Trennung des Umwälzkreises von der Wasserquelle, ein Mittel (22a) zur Umwälzung des Wassers oder der teilweise hergestellten Flüssigkeit durch das Mischgefäß (21a), und ein Mittel zur Messung der Konzentration in dem Umwälzkreis (20), um die Umwälzung anzuhalten, wenn eine entsprechende Konzentration durch eine vollständige oder teilweise Auflösung des Pulvers erreicht ist.

11. Vorrichtung nach Anspruch 10, worin das Mischgefäß auch eine geringe Menge einer Flüssigkeit, vorzugsweise Wasser, in einer solchen Menge enthält, dass eine konzentrierte Flüssigkeit aus der Flüssigkeit und dem Pulver gebildet wird.

12. Vorrichtung nach Anspruch 10 oder 11, worin das Mischgefäß (21a) eine flüssige Säure sowie das Pulver enthält.

13. Vorrichtung nach einem der Ansprüche 10 bis 12, worin der Umwälzkreis (20a) eine Heizvorrichtung (54a) umfasst.

14. Vorrichtung nach einem der Ansprüche 10 bis 13, worin der Umwälzkreis (20a) an einen Zweigkanal (54a) mit einer Dosierpumpe (56a) angeschlossen ist, der so angeordnet ist, dass er eine Säure in flüssiger Form von einem Lagergefäß (57a) zu dem Umwälzkreis pumpt.

15. Vorrichtung nach einem der Ansprüche 10 bis 14, worin der Umwälzkreis (20a) einen Sterilfilter (58a) umfasst, welcher vorzugsweise in Umwälzrichtung genau vor dem Mischgefäß (21a) angeordnet ist.

16. Vorrichtung nach einem der Ansprüche 10 bis 15, worin das Mischgefäß (21a) an den Umwälzkreis mit Hilfe von Schnellöseverbindungen (59a) angeschlossen ist, so dass es leicht abgekoppelt und stattdessen zum Beispiel an Stelle der in herkömmlicher Weise verwendeten Behälter für Dialysekonzentrat an eine Dialysemaschine angeschlossen werden kann.

17. Vorrichtung nach einem der Ansprüche 10 bis 16, worin das Mischgefäß (21a) aus einem flexiblen Kunststoffbeutel oder einer Kartusche besteht.

18. Vorrichtung nach einem der Ansprüche 10 bis 17, worin das Mittel zur Messung der Konzentration einen Leitfähigkeitsmesser (23a) umfasst.

19. Vorrichtung nach einem der Ansprüche 10 bis 18, worin der Umwälzkreis an einen ersten Flüssigkeitsanschluss (59a) des Mischgefäßes (21a) zur Entnahme des Wassers oder der teilweise hergestellten Flüssigkeit aus dem Mischgefäß sowie an einen zweiten Flüssigkeitsanschluss (59a) des Mischgefäßes (21a) zur Rückführung des Wassers oder der teilweise hergestellten Flüssigkeit zu dem Mischgefäß angeschlossen ist, wobei der erste Flüssigkeitsanschluss über dem zweiten Flüssigkeitsanschluss angeordnet ist.

20. Vorrichtung nach einem der vorhergehenden Ansprüche, worin das Mischgefäß (21a) zur Herstellung einer Dialyseflüssigkeit eine Menge in der Größenordnung von 1 kg eines Pulvergemisches von geeigneter, zum Beispiel herkömmlicher, Zusammensetzung enthält, zu welcher eine Menge in der Größenordnung von 2 dl einer Flüssigkeit zugegeben wird, zur Herstellung einer klumpenfreien, konzentrierten Flüssigkeit oder Suspension, welche vorzugsweise die für die Dialyseflüssigkeit notwendige Menge an Säure enthält.

## Revendications

1. Procédé de préparation d'un fluide à usage médical, par exemple un fluide de dialyse ou un fluide de remplacement pour hémofiltration ou un concentré pour la préparation de tels fluides, comprenant les étapes de :
préparation d'une source (1a) d'eau et mesure (23a) de la conductivité de l'eau ou du fluide partiellement préparé, pour terminaison de la recirculation lorsqu'une concentration appropriée est obtenue, d'un récipient de mélange (21a) contenant une poudre qui doit être dissoute dans la dite eau pour préparation du fluide désiré, de moyens de conduite de l'eau au dit récipient de mélange (21a), d'un circuit de recirculation (20a) incluant ce récipient de mélange (21a), et de moyens (22a) de recirculation de l'eau ou du fluide partiellement préparé à travers le dit récipient de mélange (21a),
fourniture d'eau venant de la dite source (20a),
après terminaison de la fourniture d'eau venant de la dite source (1a), recirculation de l'eau ou du fluide partiellement préparé à travers le dit récipient de mélange,
mesure (23a) de la conductivité de l'eau ou du fluide partiellement préparé dans le dit circuit de recirculation, et
arrêt de la recirculation lorsqu'une concentration appropriée est atteinte du fait de la dissolution complète ou partielle de la poudre.

2. Procédé selon la revendication 1, comprenant en outre l'étape de remplissage du récipient de mélange avec un excès de concentré en poudre, de préférence en excès de la quantité requise pour une pluralité de doses de patients individuels.

3. Procédé selon une quelconque des revendications précédentes, comprenant en outre l'étape de filtration de l'eau ou du fluide partiellement préparé dans le circuit de recirculation.

4. Procédé selon une quelconque des revendications précédentes, comprenant en outre l'étape d'introduction d'acide dans le dit circuit de recirculation (20a).

5. Procédé selon une quelconque des revendications précédentes, dans lequel la dite poudre est sous forme sèche.

6. Procédé selon les revendications 1 à 4, dans lequel la dite poudre est sous la forme d'un liquide concentré ou d'une boue obtenue par addition d'une petite quantité de liquide.

7. Procédé selon une quelconque des revendications précédentes, dans lequel la dite poudre comprend un acide sous forme de liquide ou de poudre sèche.

8. Procédé selon une quelconque des revendications précédentes, comprenant en outre l'étape de déconnexion du récipient de mélange (21a) du circuit de recirculation afin de connecter le récipient de mélange (21a) à une machine de dialyse.

9. Procédé selon une quelconque des revendications précédentes, comprenant en outre l'étape de recirculation de la dite solution partiellement préparée, à partir d'une première connexion de fluide du dit récipient de mélange, via le conduit de recirculation, jusqu'à une deuxième connexion de fluide du dit récipient de mélange, de sorte que la dite première connexion de fluide est placée au-dessus de la dite deuxième connexion de fluide.

10. Appareil pour la préparation d'un fluide à usage médical, par exemple un fluide de dialyse ou un fluide de remplacement pour hémofiltration ou un concentré pour la préparation de tels fluides, incluant une source (1a) d'eau et un récipient de mélange (21a) contenant une poudre qui doit être dissoute dans la dite eau pour la préparation du fluide désiré, comprenant des moyens de conduite de l'eau au dit récipient de mélange (21), un circuit de recirculation (20a) incluant ce récipient de mélange (21a), une vanne pour déconnecter le circuit de recirculation de la dite source d'eau, des moyens (22a) de recirculation de l'eau ou du fluide partiellement préparé à travers le dit récipient de mélange (21a), et des moyens de mesure de la concentration dans le circuit de recirculation (20) pour terminer la recirculation lorsqu'une concentration appropriée est obtenue par la dissolution complète ou partielle de la poudre.

11. Appareil selon la revendication 10, dans lequel le récipient de mélange contient également une petite quantité d'un liquide, de préférence de l'eau, en une quantité telle qu'il se forme un liquide concentré comprenant le dit liquide et la poudre.

12. Appareil selon la revendication 10 ou 11, dans lequel le récipient de mélange (21a) contient un acide liquide ainsi que la dite poudre.

13. Appareil selon une quelconque des revendications 10 à 12, dans lequel le circuit de recirculation (20a) comprend un dispositif de chauffage (54a).

14. Appareil selon une quelconque des revendications 10 à 13, dans lequel le circuit de recirculation (20a) est connecté à un conduit de dérivation (54a) équipé d'une pompe de dosage (56a), qui est agencée pour pomper un acide sous forme liquide d'un récipient de stockage (57a) au circuit de recirculation.

15. Appareil selon une quelconque des revendications 10 à 14, dans lequel le circuit de recirculation (20a) comprend un filtre stérile (58a) qui est de préférence agencé dans la direction de circulation juste avant le récipient de mélange (21a).

16. Appareil selon une quelconque des revendications 10 à 15, dans lequel le récipient de mélange (21a) est connecté au circuit de recirculation au moyen de connecteurs à démontage rapide (59a) de sorte qu'il peut facilement être désaccouplé et connecté, par exemple à une machine de dialyse, au lieu du récipient de concentré de dialyse habituellement employé.

17. Appareil selon une quelconque des revendications 10 à 16, dans lequel le récipient de mélange (21a) consiste en une poche en matière plastique souple ou une cartouche.

18. Appareil selon une quelconque des revendications 10 à 17, dans lequel les dits moyens de mesure de la dite concentration comprennent un dispositif de mesure de conductivité (23a).

19. Appareil selon une quelconque des revendications 10 à 18, dans lequel le dit circuit de recirculation est connecté à une première connexion de fluide (59a) du dit récipient de mélange (21a) pour extraire l'eau ou le fluide partiellement préparé du dit récipient de mélange, et à une deuxième connexion de fluide (59a) du dit récipient de mélange (21a) pour retourner l'eau ou le fluide partiellement préparé au dit récipient de mélange, de sorte que la dite première connexion de fluide est placée au-dessus de la dite deuxième connexion de fluide.

20. Appareil selon une quelconque des revendications précédentes, dans lequel le récipient de mélange (21a) pour préparation d'un fluide de dialyse contient une quantité de l'ordre de 1kg de mélange en poudre d'une composition appropriée, par exemple usuelle, à laquelle une quantité de l'ordre de 2 dl d'un liquide est ajoutée pour la préparation d'un liquide concentré ou d'une boue exempte de morceaux, qui contient de préférence la quantité nécessaire d'acide pour le fluide de dialyse.
